# EUROPÄISCHE PATENTANMELDUNG

(11) **EP 2 545 940 A1**
(43) Veröffentlichungstag der Anmeldung: **16.01.2013**
(21) Anmeldenummer: 11173982.7
(22) Anmeldetag: 14.07.2011
(51) Int. Cl.: A61K 47/10, A61K 9/00

(54) **Parenterale Zusammensetzungen**

(71) Anmelder: hameln rds gmbh, 31789 Hameln (DE)
(72) Erfinder: Smahovsky, Vendelin, 902 01 Pezinok (SK); Muranyi, Andrej, 85104 Bratislava (SK); Psenkova, Jana, 91701 Trnava (SK); Snauko, Marian, 90001 Modra (SK)

(57) **Zusammenfassung**

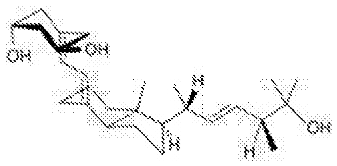

Es wird die Herstellung einer Formulierung beschrieben, die eine parenterale Verabreichung eines Vitamin-D analogons mit geringeren Gehalten an organischen Lösungsmitteln ermöglicht.

## Beschreibung

### Gebiet der Erfindung

Die vorliegende Erfindung offenbart eine neue Formulierung für Vitamin D-Analoga und deren Herstellung.

### STAND DER TECHNIK

Paricalcitol ((1 R, 3R, 7E, 17β) -17 - [(1 R, 2E, 4S)-5-Hydroxy-1 ,4,5-trimethylhex-2-en-1-yl] -9,10-secoestra- 5,7-Dien-1 ,3-diol), eine biologisch aktive Vitamin-D-Verbindung, wurde zum ersten Mal in EP0387077 offenbart.

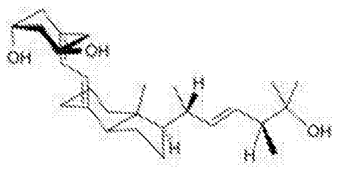

Der Wirkstoff wird parenteral oder oral verabreicht. Die derzeit verfügbaren Produkte sind als eine 5µg/ml Lösung zur parenteralen Anwendung und eine Kapsel unter dem Handelsnamen Zemplar^{®} erhältlich.

Es gibt verschiedene Möglichkeiten parenterale Lösungen von pharmazeutischen Wirkstoffen zu stabilisieren. Dabei ist es ein ständiges Ziel der galenischen Entwicklung, wirksame Formulierungen zu entwickeln, die Vorteile bieten bei der Herstellung, Verarbeitung und der Sicherheit für den Patienten. Für die Entwicklung einer parenteralen Formulierung ergeben sich dabei besondere Hürden.

Hindernisse, die bei der Entwicklung von parenteralen Lösungen zu überwinden sind, sind z. B. Sauerstoff-Empfindlichkeit, Lichtempfindlichkeit und thermische Instabilität der Lösungen. In dem besonderen Fall der Vitamin D-Verbindungen mit einer eher geringen Löslichkeit in wässrigem Medium, ist es auch das Ziel eine Auflösung des API in ausreichender Menge zu gewährleisten.

Im Stand der Technik gibt es verschiedene Hilfsstoffe, die eingesetzt werden um parenterale Lösungen zu stabilisieren. Dies sind zum Beispiel Antioxidantien, Puffer, Komplexbildner und Lösungsvermittler.

Bei stark hydrophoben Wirkstoffen werden verschiedene Lösungsvermittler und organische Lösungsmittel als Hilfsmittel im Stand der Technik erwähnt. Allerdings induzieren einige dieser Hilfsmittel unerwünschte Nebenwirkungen wie Kopfschmerzen, Erbrechen, Leberschäden oder Hämolyse.

In EP107346781 wird eine Formulierung beschrieben, die mit 30% Propylenglykol und 20% Ethanol etwa 50 % organische Lösungsmittel verwendet.

Der Stand der Technik in EP107346781 ist als Auszug im folgenden Absatz zitiert "An adequate amount of paracalcin was weighed and added to 10 ml of cosolvent contained in a 10 ml stoppered glass test tube. Two samples were prepared for each cosolvent composition. The test tubes containing samples were shaken in a 25°C reciprocal shaking water bath at 100 rpm. Upon complete dissolution, an aliquot was filtered through a 0.45 µm syringe filter and the filtrate diluted 1:1 with 50% methanol. The resulting diluted material was measured for the content of paracalcin. Table 1 shows the results of concentration of paracalcin in the listed cosolvent systems.

**TABLE 1**

| Ethanol | Propylene glycol | Water | Paracalcin (µg/ml) |
|---|---|---|---|
| 000 | 0506 | 0494 | 1421 |
| 000 | 0302 | 0698 | 058 |
| 0100 | 0257 | 0643 | 288 |
| 0199 | 0207 | 0594 | 1594 |
| 0 201 | 0308 | 0491 | 72 90 |
| 0 299 | 000 | 0701 | 682 |
| 0347 | 0102 | 0551 | 119 37 |
| 0498 | 000 | 0502 | 601 63 |

Es ist ersichtlich, dass nur Lösungen mit einem Minimum von 20% Ethanol oder einem Gehalt von nichtwässrigen Lösungsmitteln von insgesamt 50% und mehr zu einer Lösung mit der gewünschten Konzentration von mindestens 5 µg/ml führen. Da aber hohe Mengen an Ethanol eine Gefahr für den Patienten darstellen, sollte der Gehalt möglichst auf ein Minimum reduziert werden.
Der Stand der Technik von EP107346781 lehrt entsprechend: "lt will be appreciated by those skilled in the clinical arts that the amount of organic solvent in the preferred parenteral formulations of the invention should be kept to a minimum. At the same time the requirements of manufacturing and required dosage ranges must be considered to ensure adequate solubility of the vitamin D compound in the present formulations."

Basierend auf dem nächstliegenden Stand der Technik ist also das zu lösende Problem, das Niveau von nicht-wässrigen Lösungsmitteln in Formulierungen für die parenterale Verabreichung von Vitamin D Analoga zu minimieren, und insbesondere den Gehalt von Ethanol in der Lösung zu reduzieren.

### Beschreibung der Erfindung

Die vorliegende Erfindung beschreibt eine sterile wässrige pharmazeutische Zusammensetzung, die Paricalcitol oder andere Vitamin D-Analoga enthält. Die für die parenterale Verabreichung vorgesehene Formulierung enthält weniger organische Lösungsmittel und vor allem weniger Ethanol als im Stand der Technik bekannt. Es wird außerdem die Herstellung solcher Lösungen beschrieben.

### Detaillierte Beschreibung der Erfindung

Aus dem Stand der Technik ist bekannt, dass eine Lösung mit einem Gehalt von mindestens 20% Ethanol oder einem Gesamtgehalt von mindestens 50% nicht-wässrigen Lösungsmitteln für die parenterale Verabreichung von Vitamin D Analoga eine stabile Formulierung ergibt. Ziel dieser Erfindung war es, die Löslichkeit des Wirkstoffs mit einem geringeren Gehalt an Ethanol oder mit niedrigerem Gesamtgehalt an nicht-wässrigen Lösungsmitteln zu erhöhen. Wir haben nun überraschend festgestellt, dass eine Lösung mit weniger Ethanol-Anteil und insgesamt weniger nicht-wässrigen Lösungsmittel als bekannt mit Hilfe einer unten näher beschriebenen Art der Herstellung ebenfalls zu einer stabilen Lösung von Paricalcitol mit der erforderlichen Konzentration des Wirkstoffs führen kann.

Obwohl die vorliegende Erfindung im Wesentlichen die Verwendung von Paricalcitol ((1R, 3R, 7E, 17β) -17 - [(1R, 2E, 4S)-5-Hydroxy-1 ,4,5-trimethylhex-2-en-1 -yl] -9,10-secoestra-5 ,7-dien-1 ,3-diol) beschreibt, können die erfindungsgemäßen Formulierungen auch für andere Vitamin D-Analoga wie Calcitriol verwendet werden. Unterschiedliche Lösungsvermittler wurden getestet, um den Ethanol-Gehalt auf weniger als 20% zu reduzieren, oder den Gesamtgehalt an nicht-wässrigen Lösungsmitteln auf weniger als 50% zu erniedrigen.

Für die Auswertung der Löslichkeit in verschiedenen Formulierungen wurden Chargen mit einem Paricalcitol-Gehalt von 20 µg/ml und einem Volumen von 100 ml hergestellt. Um eine größtmögliche Konzentration des Wirkstoffs in der Lösung zu erzielen, wurde der Wirkstoff zuerst in Ethanol gelöst, und anschließend mit anderen Hilfsstoffen, Lösungsmitteln oder Wasser vermischt.
Die Formulierung der Lösung wurde in den folgenden Schritten durchgeführt:
a. Ethanol vorlegen, falls verwendet
b. Vermischen des Wirkstoffs mit Ethanol (nur im Batch-4 API nach Propylenglykol hinzufügen), und rühren, bis eine klare Lösung entsteht
c. Kombinieren der alkoholischen Lösung mit Propylenglykol
d. Rühren, bis eine klare Lösung entsteht
e. Vermischen der Lösung mit WFI

**Tabelle 2**

| Batch | Formulierung | Gehalt [µg/ml] |
|---|---|---|
| 1 | Ethanol- 20 % v/v Propylenglykol- 30 % v/v | 18.4 |
| 2 | Ethanol- 15 % v/v Propylenglykol- 25 % v/v | 18.2 |
| 3 | Ethanol- 10 % v/v Propylenglykol- 30 % v/v | 17.9 |
| 4 | Propylenglykol - 30 % v/v | 2.4 |
| 5 | Ethanol- 20 % v/v Propylenglykol- 10 % v/v | 7.5 |
| 6 | Ethanol- 20 % v/v | 0.4 |
| 7 | Ethanol- 30 % v/v | 7.1 |
| 8 | Ethanol- 30 % v/v Propylenglykol- 10 % v/v | 19.3 |
| 9 | Ethanol- 40 % v/v Propylenglykol- 10 % v/v | 19.5 |
| 10 | Benzylalkohol- 1.5 % v/v Tween 20- 15% w/v | 19.3 |
| 11 | Ethanol- 10 % v/v Cremophor- 5% w/v | 20.0 |
| 12 | Ethanol- 10 % v/v Tween 20- 5 % w/v PEG 300- 15% v/v | 19.7 |

50 ml von jeder Charge wurden mit ausreichend Volumen an Lösungsmitteln, Co-Lösungsmittel und Tensiden (abhängig von der konkreten Formulierung) bis zu einem Gesamtvolumen von 200 ml aufgefüllt. Jede Charge durch 0,2 µm Flurodyne ® PVDF Spritzenfilter filtriert und in 10 ml Durchstechflaschen gefüllt, Typ I, Stopfen FM 259 / 0 Omniflex ® plus.
Tabelle 3 zeigt die Ergebnisse ausgewählter Formulierungen in einer Stabilitätsstudie bei 40 °C.

**Tabelle 3**

| Gehalt Paricalcitol in ausgewählten Chargen bei 40°C / 75% RH (Start = 100 %) | | | | |
|---|---|---|---|---|
| Paricalcitol (HPLC) [%] | Start | Nach 15 Tagen | Nach 30 Tagen | Nach 60 Tagen |
| 2 | 100 | 99,9 | 99,5 | 99,7 |
| 3 | 100 | 98,4 | 101,5 | 100,2 |
| 8 | 100 | 99,8 | 102,8 | 99,8 |
| 11 | 100 | 88,6 | 80,8 | 52,0 |
| 12 | 100 | 67,3 | 47,8 | 9,9 |

Während des Tests wurde überraschend festgestellt, dass die Verwendung von Ethanol als Lösungsmittel für eine Vor-Lösung eine Formulierung mit weniger Ethanol und / oder weniger Gesamtgehalt an nicht-wässrigen Lösungsmitteln ermöglicht. Diese Lösung ist sowohl bei 40 °C als auch bei 25 °C stabil.

Ähnliche Ergebnisse werden erzielt, wenn zur ethanolischen Lösung des Wirkstoffs zunächst das WFI, und anschließend der Lösungsvermittler, oder das Cosolvent zugesetzt werden.

Wie in EP107346781 beschrieben war es nach dem Stand der Technik nicht möglich eine Lösung von Paricalcitol mit 10 % Ethanol und 30 % Propylenglycol herzustellen, die den Wirkstoff in genügend hoher Konzentration enthält.
Mit der erfindungsgemäßen Herstellung kann eine genügend hohe Konzentration von Paricalcitol in Lösungen mit geringerem Gehalt an Ethanol und einem geringeren Gesamtgehalt an organischen Lösungsmitteln erreicht werden.

Vergleicht man die erfindungsgemäßen Formulierungen mit den auf dem Markt befindlichen Formulierungen von ZEMPLAR^{®}, so konnten wir außerdem feststellen, dass die erfindungsgemäße Formulierung weniger Verunreinigungen enthält. Siehe Tabelle 4 (Test vom Oktober 2010.)

**Tabelle 4**

| | Paricalcitol 02210111 | Zemplar® 811758E07 |
|---|---|---|
| | Sterilized (121 °C/15 min) | Innovator (exp. date: 09/2011) |
| Assay (HPLC) [µg/ml] | 4.85 (97.0 %) | 4.82 (96.4%) |
| Related Susbtances [%]: | | |
| Imp. A | n.d. | n.d. |
| Imp. B | n.d. | n.d. |
| Imp. D | n.d. | n.d. |
| Imp. E | n.d. | n.d. |
| Greatest single imp. | 0.10 | 0.19 |
| Total imp. | 0.64 | 1.55 |

Nach Herstellung der Lösung kann die Lösung in Ampullen oder Vials gefüllt und abschließend sterilisiert werden.

Die Lösungen der Erfindung sind stabil gegenüber Sterilisation im Autoklaven bei 121 °C. Nach der Sterilisation sind die Proben bei Raumtemperatur für mindestens 24 Monate stabil.

Es wird vorausgesetzt, dass dem Fachmann klar ist, dass alle eingesetzten Lösungsmittel und Hilfsstoffe einer Qualität entsprechen, die für den Einsatz in einem pharmazeutischen Produkt notwendig ist.

Wenn eine wässrige Zusammensetzung organische Lösungsmittel in einem bestimmten Prozentsatz enthält, ist es klar, dass der Rest der Lösung mit Wasser in pharmazeutischer Qualität zu 100% des Volumens aufgefüllt wird.

API bezeichnet einen pharmazeutischen Wirkstoff und wird in dieser Erfindung gleichbedeutend mit den Begriffen "Paricalcitol", "Vitamin D-Verbindung" oder "Vitamin D Analogon" verwendet.

Eine Ausführungsform der Erfindung beschreibt eine stabile wässrige pharmazeutische Zusammensetzung für die parenterale Verabreichung von Vitamin D Analoga, die ein solches Vitamin-D-Analogon in einer therapeutisch wirksamen Konzentration, Wasser und ein oder mehrere organische Lösungsmittel enthält, wobei die Summe von organischen Lösungsmitteln eine Gesamtmenge von etwa 45% (v/v) nicht übersteigt und wobei die Menge an Ethanol einen Gehalt von etwa 15 % (v/v) nicht übersteigt.

In einer bevorzugten Ausgestaltung der Erfindung sind die organischen Lösungsmittel in der Zusammensetzung ausgewählt aus der Gruppe von Ethylenglykol, Propylenglykol und Ethanol.

In einer besonders bevorzugten Ausführungsform der Erfindung sind die organischen Lösungsmittel in der Zusammensetzung ausgewählt aus der Gruppe von Propylenglykol und Ethanol.

In einer Ausgestaltung der Erfindung wird die erfindungsgemäße Lösung in Durchstechflaschen oder Ampullen gefüllt. In einer bevorzugten Ausgestaltung der Erfindung wird die Lösung in Ampullen abgefüllt.

Es ist möglich, die erfindungsgemäßen Lösungen in einem Autoklaven bei 121 °C zu sterilisieren.

In einer bevorzugten Ausgestaltung enthält die erfindungsgemäße pharmazeutische Zusammensetzung für die parenterale Verabreichung den Wirkstoff Paricalcitol

In einer bevorzugten Ausgestaltung der Erfindung enthält die Zusammensetzung den Wirkstoff in einer Menge von etwa 2 bis 10 µg / ml.

In einer besonders bevorzugten Ausgestaltung der Erfindung enthält die Zusammensetzung den Wirkstoff in einer Menge von etwa 5 µg / ml.

Eine besonders bevorzugte Ausgestaltung der Erfindung enthält in der pharmazeutischen Zusammensetzung für die parenterale Verabreichung Paricalcitol in einer Konzentration von 5 µg / ml, Ethanol mit einem Gehalt von etwa 10% (v/v), Propylenglykol mit einem Gehalt von etwa 30% (v/v) und Wasser für Injektionszwecke.

Ein Verfahren zur Herstellung einer erfindungsgemäßen pharmazeutischen Formulierung, die eine therapeutisch wirksame Menge von Paricalcitol enthält umfasst die folgenden Schritte:
a. Auflösen des API in Ethanol und rühren, bis eine klare Lösung entsteht
b. Kombination mit Propylenglykol und Rühren, bis eine klare Lösung entsteht
c. Kombination mit Wasser und Rühren, bis eine klare Lösung entsteht

### Beispiele

Die Konzentration der Vitamin D-Verbindung in den Formulierungen ist variabel. Die Beispiele verwenden daher den Ausdruck "die notwendige Menge". Diese Menge kann im Bereich der therapeutischen Dosis, die zwischen 2 und 10 µg/ml liegt, variieren. Die bevorzugte Konzentration beträgt 5µ/ml, während die Tabelle 5 (siehe unten) zeigt, dass auch andere Konzentrationen möglich sind.

### Beispiel 1 Löslichkeit von Vitamin D-Verbindungen

Ein Gefäß wurde mit einem Rührer ausgestattet und der Alkohol vorgelegt. Die notwendige Menge an Vitamin D-Verbindung wurde unter Rühren zugefügt und weiter gerührt, bis sich eine klare Lösung bildete. Anschließend wurde das Glykol oder Tensid zur Lösung zugegeben und das Rühren langsam fortgesetzt, bis eine klare Lösung entstand. Schließlich wurde die Lösung bis zum Endvolumen mit WFI unter ständigem Rühren aufgefüllt. Das Rühren wurde fortgesetzt, bis eine klare Lösung entstand. Die Lösung wurde durch einen 0,22 µm Spritzenfilter filtriert und in Ampullen abgefüllt.

Der Gehalt an API wurde mit Hilfe einer HPLC-Methode bestimmt.

Für Charge vier, wurde zunächst Propylenglykol vorgelegt, dann der API hinzugefügt.

Die folgenden Lösungen wurden auf diese Weise hergestellt:

**Tabelle 5**

| Charge | Formulierung | Gehalt [µg/ml] |
|---|---|---|
| 1 | Ethanol- 20 % v/v Propylenglykol- 30 % v/v | 18.4 |
| 2 | Ethanol- 15 % v/v Propylenglykol- 25 % v/v | 18.2 |
| 3 | Ethanol- 10 % v/v Propylenglykol- 30 % v/v | 17.9 |
| 4 | Propylenglykol - 30 % v/v | 2.4 |
| 5 | Ethanol- 20 % v/v Propylenglykol- 10 % v/v | 7.5 |
| 6 | Ethanol- 20 % v/v | 0.4 |
| 7 | Ethanol- 30 % v/v | 7.1 |
| 8 | Ethanol- 30 % v/v Propylenglykol- 10 % v/v | 19.3 |
| 9 | Ethanol- 40 % v/v Propylenglykol- 10 % v/v | 19.5 |
| 10 | Benzylalkohol- 1.5 % v/v Tween 20- 15% w/v | 19.3 |
| 11 | Ethanol- 10 % v/v Cremophor- 5% w/v | 20.0 |
| 12 | Ethanol- 10 % v/v Tween 20- 5 % w/v PEG 300- 15% v/v | 19.7 |

Nach der Messung des Gehalts wurden ausgewählte Lösungen mit den entsprechenden Lösungsmittelkombinationen auf ein Viertel verdünnt und die Lösungen auf Stabilität geprüft.

### Beispiel 2 Herstellung von Paricalcitol Lösung in Ampullen

In einem geeigneten Gefäß wurde bei Raumtemperatur Ethanol vorgelegt und die notwendige Menge an Paricalcitol unter Rühren zugegeben, bis sich eine klare Lösung bildete. Das Propylenglykol wurde zu der Lösung gegeben und solange gerührt, bis sich eine klare Lösung bildete. Schließlich wurde die Lösung unter ständigem Rühren bis zum Endvolumen mit WFI (das mit Stickstoff gespült wurde) aufgefüllt. Das Rühren wurde fortgesetzt, bis eine klare Lösung entstand. Die Lösung wurde mit Stickstoff begast. Die Lösung wurde durch ein 0,22 µm Nylon-Filter filtriert und in Ampullen von 1 und 2 ml (Ampullen wurden vor und nach dem Befüllen mit Stickstoff bedeckt) gefüllt, versiegelt und autoklaviert für 15 Minuten bei 121 ° C

## Patentansprüche

1. Stabile wässrige pharmazeutische Zusammensetzung für die parenterale Verabreichung von Vitamin D Analoga, die ein solches Vitamin-D-Analogon in einer therapeutisch wirksamen Konzentration, Wasser und ein oder mehrere organische Lösungsmittel enthält, wobei die Summe von organischen Lösungsmitteln eine Gesamtmenge von etwa 45% (v/v) nicht übersteigt und wobei die Menge an Ethanol einen Gehalt von etwa 15% (v/v) nicht übersteigt.

2. Zusammensetzung nach Anspruch 1, wobei die organischen Lösungsmittel ausgewählt sind aus der Gruppe von Ethylenglykol, Propylenglykol und Ethanol.

3. Zusammensetzung nach Anspruch 1, wobei die organischen Lösungsmittel ausgewählt sind aus der Gruppe von Propylenglykol und Ethanol.

4. Zusammensetzung nach einem der Ansprüche 1 - 3, wobei die Lösung in Durchstechflaschen oder in Ampullen gefüllt wird.

5. Zusammensetzung nach Anspruch 4, wobei die Lösung in einem Autoklaven bei 121 °C sterilisiert wird.

6. Zusammensetzung nach einem der vorgenannten Ansprüche, wobei die Vitamin D-Verbindung Paricalcitol ist.

7. Zusammensetzung nach einem der vorgenannten Ansprüche, die den Wirkstoff in einer Konzentration von etwa 2 bis 10 µg / ml enthält.

8. Pharmazeutische Zusammensetzung für die parenterale Verabreichung nach dem oben genannten Ansprüchen enthaltend Paricalcitol in einer Konzentration von 5µg/ml, Ethanol mit etwa 10% (v / v), Propylenglykol mit etwa 30% (v / v) und Wasser für Injektionszwecke.

9. Verfahren zur Herstellung einer pharmazeutischen Zusammensetzung, die eine therapeutisch wirksame Menge von Paricalcitol enthält, das folgende Schritte beinhaltet:
a. Auflösen des API in Ethanol und rühren, bis eine klare Lösung entsteht
b. Kombination mit Propylenglykol und Rühren, bis eine klare Lösung entsteht
c. Kombination mit Wasser und Rühren, bis eine klare Lösung entsteht
